# NOUVEAU FASCICULE DE BREVET EUROPEEN

(11) **EP 2 033 627 B2**
(45) Date de publication et mention de la décision concernant l'opposition: **02.08.2023**
(45) Mention de la délivrance du brevet: 18.04.2018
(21) Numéro de dépôt: 08163529.4
(22) Date de dépôt: 02.09.2008
(51) Int. Cl.: A61K 8/99, A61Q 19/00, A61P 17/00

(54) **Utilisation d'un lysat de Bifidobacterium species pour le traitement de peaux sensibles**
Einsatz eines Lysats einer Bifidobakteriengattung zur Behandlung von sensiblen Hauttypen
Use of a Bifidobacterium species lysate for treating sensitive skin

(30) Priorité: 04.09.2007 FR 0757352
(43) Date de publication de la demande: 11.03.2009
(73) Titulaire: L'OREAL, 75008 Paris (FR)
(72) Inventeur: Breton, Lionel, 78000, VERSAILLES (FR); Gueniche, Audrey, 92500, RUEIL MALMAISON (FR); Castiel, Isabelle, 06200, NICE (FR)
(74) Mandataire: Cabinet Nony

(56) Documents cités:
- EP-A- 0 043 128
- EP-A- 1 731 137
- FR-A- 2 876 029
- US-A1- 2006 002 910
- GUENICHE et al.: "Bifidobacterium longum lysate, a new ingredient for reactive skin", Experimental Dermatology, vol. 19, 2009, pages e1-e8,
- BAU et al.: "Substancias funcionales (III). Description de los principios activos cosmeticos", Farmacia Practica, 2001, pages 147-150,
- Biochemical Engineering Journal; Vol.33; 2007, pp. 94-98
- Water research, VoL 45, 2011, pp 2775-2781

## Description

La présente invention vise à proposer un nouvel agent plus particulièrement utile pour la prévention et/ou le traitement de troubles cutanés indésirables et notamment utiles pour la prévention et/ou le traitement des troubles cutanés indésirables susceptibles de se manifester au niveau des peaux qualifiées de « peaux sensibles ».

D'une manière générale, les troubles cutanés indésirables peuvent se manifester sous deux aspects, à savoir sensations d'inconfort ressenties au niveau de la peau et/ou apparition de signes cutanés visibles.

Pour ce qui est des sensations d'inconfort, il peut typiquement s'agir de démangeaisons, d'échauffement, de sensations de picotement, et/ou de tiraillement. A titre représentatif, des signes cutanés visibles on peut notamment citer les prurits, les dartres, les érythèmes et/ou les rougeurs.

Ces troubles cutanés sont plus fréquents dans les zones les plus exposées de l'organisme, à savoir les mains, les pieds, le visage, et le cuir chevelu.

Cette réactivité cutanée se traduit classiquement par la manifestation de signes d'inconfort en réponse à la mise en contact du sujet avec un élément déclenchant qui peut avoir diverses origines.

Ces réactions cutanées peuvent survenir notamment sur des zones soumises à certains gestes d'hygiène quotidienne ou fréquemment renouvelés tels que le rasage, l'épilation, la détersion par des produits de toilette ou des produits ménagers, l'application d'adhésifs (pansements, patchs, fixation de prothèses) ou dans le cas de gestes sportifs, professionnels ou simplement liés au mode de vie et à l'utilisation de vêtements, d'outils ou d'équipements générant des frottements localisés à la prise de certains aliments. Elles peuvent également être amplifiées par des variations de température, le vent et du stress psychologique.

On sait en outre que le risque de manifestation de ces réactions cutanées est exacerbé dans le cas des peaux sensibles.

Ainsi, l'apparition des signes d'inconfort, qui apparaissent dans les minutes qui suivent la mise en contact du sujet avec l'élément déclenchant, est une des caractéristiques essentielles des peaux sensibles. Il s'agit principalement de sensations dysesthésiques qui sont des signes neurosensoriels. On entend par sensations dysesthésiques, des sensations plus ou moins douloureuses ressenties dans une zone cutanée comme les picotements, fourmillements, démangeaisons ou prurits, échauffements, inconforts, tiraillements, etc. Ces signes subjectifs existent le plus souvent en l'absence de signes cliniques visibles tels que les desquamations. On sait aujourd'hui que ces réactions d'intolérance cutanée sont notamment liées à une libération de neuropeptides par les terminaisons nerveuses de l'épiderme et du derme.

Par opposition aux peaux qualifiées d'allergiques, la réactivité d'une peau sensible ne relève pas d'un processus immunologique c'est-à-dire ne se produit pas en réponse à la présence d'un allergène.

De plus, son mécanisme de réponse est dit "aspécifique". Elle est, à ce titre, à distinguer des peaux manifestant des réactions inflammatoires et allergiques de type dermatose, eczéma, et/ou ichtyoses, et vis-à-vis desquelles un certain nombre de traitements ont déjà été proposés.

Pour des raisons évidentes, il serait avantageux de disposer de compositions aptes à prévenir et/ou traiter ces manifestations de troubles cutanés indésirables.

La présente invention a précisément pour objet de répondre à ce besoin.

De manière inattendue, les inventeurs ont constaté que certains microorganismes du genre *Bifidobacterium species* pouvaient s'avérer efficaces, pour la prévention et/ou le traitement de troubles cutanés indésirables au niveau des peaux sensibles sous réserve qu'ils soient mis en oeuvre sous la forme spécifique d'un lysat formé de tous les constituants biologiques intracellulaires et des constituants des parois et membranes cellulaires.

Le document WO 02/28402 décrit que des microorganismes probiotiques peuvent avoir un effet bénéfique dans la régulation de réactions d'hypersensibilité cutanée comme les réactions inflammatoires et allergiques qui relèvent d'un processus immunologique. Il est également rapporté dans "Probiotics in the managment of atopic eczema, Clinical and Experimental Allergy 2000", Volume 30, pages 1604-1610, une étude concernant l'effet de probiotiques sur les mécanismes immunitaires infantiles comme par exemple la dermatite atopique. Le document US 5 756 088, décrit un régime alimentaire, comprenant notamment un acide gras polyinsaturé et/ou de la biotine, et un Bifidobacterium, ayant des effets prophylactiques et thérapeutiques sur les dermatoses animales.

Plus récemment, le document EP 1 609 463 décrit des compositions associant un microorganisme probiotique à un cation minéral pour le traitement des peaux sensibles et le document EP 1 642 570 propose à cette même fin des mélanges spécifiques de microorganismes probiotiques. Quant au document PCT/FR2006/050768, il propose pour le traitement des peaux sensibles associées à une peau sèche, une association d'un microorganisme probiotique avec un acide gras polyinsaturé et/ou ester d'acide gras polyinsaturé.

Le document EP 1 731 137, pour sa part, décrit la mise en oeuvre d'un mélange *Lactobacillus paracaseï* ou *caseï* et *Bifidobacterium lactis,* utile pour le traitement des peaux sensibles et/ou sèches.

De même, FR 2 876 029 divulgue l'utilisation d'un mélange *Bactobacillus paracaseï* ou *caseï* et *Bifïobacterium longum* pour le traitement des peaux sensibles et/ou sèches.

Toutefois, aucun de ces documents ne considère la mise en oeuvre d'un actif conforme à la présente invention, sous forme d'un lysat.

On connaît de EP 0 043 128, la mise en oeuvre d'un lysat de microorganisme tel que le lysat Repair Complex CLR, utile, pour sa part, sur le processus de réparation de l'ADN des cellules de la peau.

Ainsi, le présent texte décrit un lysat d'au moins un microorganisme du genre *Bifïdobacterium species* en une quantité efficace, pour son utilisation pour prévenir et/ou traiter un trouble cutané au niveau d'une peau sensible, ledit lysat étant formé de tous les constituants biologiques intracellulaires et des constituants des parois et membranes cellulaires. Selon un premier de ses aspects, la présente invention concerne ainsi l'utilisation cosmétique non-thérapeutique d'une quantité efficace d'au moins un lysat d'au moins un microorganisme du genre *Bifidobacterium* pour prévenir et/ou diminuer les sensations d'inconfort cutané au niveau d'une peau sensible, ledit lysat étant formé de tous les constituants biologiques intracellulaires et des constituants des parois et membranes cellulaires, lesdites sensations d'inconfort cutané étant choisies parmi les démangeaisons, les échauffements, les sensations de picotement et/ou de tiraillement, ledit lysat étant administré par voie topique.

En effet, les inventeurs de la présente invention ont mis en évidence que le lysat de microorganisme du genre *Bifïdobacterium sp* pouvait avoir une action directe sur les fibres nerveuses en limitant la libération de neuromédiateurs tels que les CGRP, au niveau cutané.

Ainsi, la présente invention repose sur la mise en évidence par les inventeurs de l'aptitude d'un lysat conforme à l'invention, à inhiber l'activité des neurones sensitifs après une irritation, et par la même, les propriétés apaisantes de ce lysat.

Au sens de l'invention, l'expression « trouble cutané » couvre les sensations d'inconfort, chez un sujet de peau sensible, ainsi que les signes cutanés visibles et inesthétiques temporaires susceptibles d'affecter ce même sujet.

Comme précisé précédemment, les sensations d'inconfort peuvent être des démangeaisons, échauffements, picotements et/ou tiraillements.

Ces troubles indésirables concernent tout particulièrement les personnes dites à peau sensible, qui possèdent un seuil de réactivité abaissé du à une hyperactivité neurogène ; ces peaux vont donc présenter ces sensations et ces signes cliniques beaucoup plus rapidement et fréquemment que les autres types de peaux.

Le présent texte décrit une quantité efficace d'au moins un lysat d'au moins un microorganisme, du genre *Bifidobacterium,* pour son utilisation citée ci-dessus, ledit lysat de microorganisme étant destiné à prévenir et/ou traiter les manifestations de sensations dysesthétiques au niveau des peaux sensibles.

La présente invention vise également une utilisation citée ci-dessus, dans laquelle ledit lysat de microorganisme est destiné à prévenir et/ou traiter les manifestations de sensations dysesthétiques au niveau des peaux sensibles.

Le texte décrit également une quantité efficace d'au moins un lysat d'au moins un microorganisme du genre Bifidobacterium pour son utilisation citée ci-dessus, ledit lysat de microorganisme étant destiné à inhiber la libération des neuromédiateurs cutanés.

Selon un autre de ses aspects, le présent brevet décrit l'utilisation d'une quantité efficace d'au moins un lysat d'au moins un microorganisme du genre *Bifidobacterium* pour la préparation d'une composition, notamment cosmétique ou dermatologique, destinée à prévenir et/ou traiter des troubles cutanés en particulier au niveau des peaux sensibles.

Selon un autre de ses aspects, le présent brevet décrit un procédé, notamment cosmétique, pour prévenir et/ou traiter une peau sensible chez un sujet comprenant au moins l'administration, notamment topique ou orale audit sujet, d'au moins une quantité efficace d'au moins un lysat d'au moins un microorganisme du genre *Bifidobacterium species* et/ou fraction de celui-ci.

Selon encore un autre de ses aspects, l'invention a pour objet une composition, cosmétique ou dermatologique, utile pour traiter et/ou prévenir un trouble cutané au niveau d'une peau sensible par voie topique, comprenant, dans un milieu physiologiquement acceptable, au moins une quantité efficace d'au moins un lysat d'au moins un microorganisme du genre *Bifidobacterium*, ledit lysat étant formé de tous les constituants biologiques intracellulaires et des constituants des parois et membranes cellulaires, en association à au moins un composé susceptible de provoquer une irritation cutanée et comprenant en outre un microorganisme probiotique annexe et/ou un lysat d'au moins un microorganisme probiotique annexe.

Par « quantité efficace », on entend au sens de la présente invention une quantité suffisante pour obtenir l'effet attendu.

Au sens de la présente invention, « prévenir » signifie diminuer le risque de manifestation d'un phénomène.

Au sens de la présente invention, le terme « traiter » signifie suppléer à un dysfonctionnement physiologique et plus généralement à diminuer, voire supprimer un désordre indésirable et dont la manifestation est notamment une conséquence de ce dysfonctionnement.

Comme précisé précédemment, les compositions, et utilisations selon l'invention, s'avèrent ainsi tout particulièrement efficaces pour protéger et/ou traiter les peaux sensibles.

Une peau sensible est différente d'une peau allergique. Sa réactivité ne relève pas d'un processus immunologique et se traduit généralement uniquement par des sensations dysesthésiques.

Pour des raisons évidentes, l'absence de signes visibles rend difficile le diagnostic de peau sensible. Le plus souvent ce diagnostic repose sur l'interrogatoire du patient. Cette symptomatologie a en outre pour intérêt de permettre de différencier la peau sensible associée ou non à une peau sèche, de l'irritation ou de l'allergie de contact pour lesquelles il existe en revanche des signes inflammatoires visibles.

En conséquence, la mise au point de produits "peaux sensibles" a nécessité de disposer d'outils d'évaluation de la réaction sensorielle de la peau. Les premiers outils se sont inspirés dès leur conception de la caractéristique essentielle des peaux sensibles à savoir la présence de signes d'inconfort induits par une application topique. Ainsi, le « stinging test » à l'acide lactique a été le premier test proposé. Il est réalisé par relevé des sensations de picotements rapportées par un volontaire après application d'une solution d'acide lactique à 10 % sur les ailes du nez. Les sujets rapportant des sensations modérées ou fortes de picotements sont appelées « stingers » et considérés comme étant à peau sensible. En raison de cette sensibilité cutanée à l'application topique de produit, ces sujets sont alors sélectionnés pour tester des produits dits peaux sensibles. Plus récemment, pour activer spécifiquement les terminaisons nerveuses périphériques, impliquées dans l'inconfort et appelées nocicepteurs, récemment identifiées comme étant impliquées dans la peau sensible, de nouveaux tests ont été proposés qui utilisent précisément d'autres inducteurs d'inconfort comme la capsaïcine.

Ce second type de test, décrit dans la demande EP 1 374 913, constitue également un autre outil particulièrement utile pour le diagnostic des peaux sensibles.

Au sens de la présente invention, l'expression « peaux sensibles » couvre les peaux irritables et les peaux intolérantes.

Une peau intolérante est une peau qui réagit au moins par des sensations d'échauffement, de tiraillements, de fourmillements et/ou de rougeurs, à différents facteurs tels que l'application de produits cosmétiques ou dermatologiques ou de savon. En général, ces signes sont associés à un érythème et à une peau hyper-séborrhéique ou acnéique, voire même rosacéiforme, avec ou sans dartres.

Une peau irritable est une peau qui réagit par un prurit, c'est-à-dire par des démangeaisons ou par des picotements, à différents facteurs tels que l'environnement, les émotions, les aliments, le vent, les frottements, le rasoir, l'eau dure à forte concentration de calcaire, les variations de température, l'humidité ou la laine.

Il est entendu que ces sensations peuvent parallèlement être associées à la manifestation de signes cutanés. Ainsi, d'une manière générale, ce type de peau peut être associé à une sécheresse cutanée avec ou sans dartres ou à une peau qui présente un érythème. La sécheresse cutanée se manifeste pour l'essentiel par une sensation de tiraillements et/ou de tension. Celle-ci est aussi rugueuse au toucher et apparaît couverte de squames. Lorsque la peau est légèrement sèche, ces squames sont abondantes mais peu visibles à l'oeil nu. Elles sont de moins en moins nombreuses mais de plus en plus visibles à l'oeil nu lorsque ce désordre s'aggrave.

Dans le cadre de la présente invention, les peaux sensibles considérées ne manifestent pas de caractère inflammatoire.

### Lysat de Microorganisme(s)

Comme précisé précédemment, selon l'invention, on met en oeuvre au moins un lysat d'au moins un microorganisme du genre *Bifidobacterium* à titre d'actifs. Un lysat désigne communément un matériau obtenu à l'issue de la destruction ou dissolution de cellules biologiques par un phénomène dit lyse cellulaire provoquant ainsi la libération des constituants biologiques intracellulaires naturellement contenus dans les cellules du microorganisme considéré.

Au sens de la présente invention, le terme lysat est utilisé pour désigner l'intégralité du lysat obtenu par lyse du microorganisme concerné.

Le lysat mis en oeuvre est donc formé des constituants biologiques intracellulaires et des constituants des parois et membranes cellulaires.

Plus précisément, il contient la fraction cytoplasmique cellulaire renfermant les enzymes tels que la déhydrogénase d'acide lactique, les phosphatases, les phosphokétolases, transaldolases et les métabolites. A titre illustratif, les constituants des parois cellulaires sont notamment le peptidoglycane, la muréine ou mucopeptide et l'acide teichoique et les constituants des membranes cellulaires sont composés de glycérophospholipides.

Cette lyse cellulaire peut être accomplie par différentes technologies, telles que par exemple choc osmotique, choc thermique, par ultrasons, ou encore sous contrainte mécanique de type centrifugation par exemple.

Plus particulièrement, ce lysat peut être obtenu selon la technologie décrite dans le brevet US 4 464 362 et notamment selon le protocole suivant.

Le microorganisme considéré de type *Bifidobacterium species* est cultivé anaérobiquement dans un milieu de culture adéquat, par exemple selon les conditions décrites dans les documents US 4 464 362 et EP 43 128. Lorsque la phase stationnaire du développement est atteinte, le milieu de culture peut être inactivé par pasteurisation, par exemple à une température de 60 à 65 °C pendant 30 mn. Les microorganismes sont alors recueillis par une technique de séparation conventionnelle par exemple filtration membranaire, centrifugation et remis en suspension dans une solution NaCl physiologique stérile.

Le lysat peut être obtenu par désintégration aux ultras-sons d'un tel milieu afin d'en libérer les fractions cytoplasmiques, les fragments de paroi cellulaire et les produits issus du métabolisme. Puis tous les composants dans leur distribution naturelle sont ensuite stabilisés dans une solution aqueuse faiblement acide.

On obtient ainsi généralement une concentration de l'ordre de 0,1 à 50 %, en particulier de 1 à 20 % et notamment environ 5 % en poids en matière(s) active(s) par rapport au poids total du lysat.

Le lysat peut être mis en oeuvre sous différentes formes, sous la forme d'une solution ou sous une forme pulvérulente.

Le microorganisme appartenant au genre *Bifidobacterium* est plus particulièrement choisi parmi les espèces : *Bifidobacterium longum Bifidobacterium bifidum, Bifidobacterium breve, Bifidobacterium animalis, Bifidobacterium lactis, Bifidobacterium infantis, Bifidobacterium adolescentis* ou *Bifidobacterium pseudocatenulatum* et leurs mélanges.

Convient tout particulièrement à l'invention, l'espèce *Bifidobacterium longum.*

Le terme fraction désigne plus particulièrement un fragment dudit microorganisme ou une partie dudit lysat de microorganisme, doté d'une efficacité pour le traitement des épidermes secs par analogie audit microorganisme entier ou audit lysat dans sa totalité.

Le produit commercialisé sous la dénomination Repair Complex CLR^{®} par la société K. RICHTER GmbH et qui est formé d'un lysat inactivé de l'espèce *Bifidobacterium longum,* entre dans le cadre de l'invention.

L'actif formant le lysat appartenant au genre *Bifidobacterium species* peut être formulé à raison d'au moins 0,0001 % (exprimé en poids sec), en particulier à raison de 0,001 à 20% et plus particulièrement à raison de 0,001 à 2 % en poids par rapport au poids total du support ou de la composition le contenant.

Avantageusement, selon l'invention, on met en oeuvre un lysat d'une seule espèce de microorganisme, notamment probiotique.

Selon une variante de l'invention, ce lysat peut être mis en oeuvre en association avec un autre microorganisme ou lysat de celui-ci ou fraction de ceux-ci.

Ainsi, selon un mode de réalisation particulier de l'invention, les compositions selon l'invention peuvent en outre contenir un lysat annexe d'au moins un microorganisme annexe, notamment de type probiotique et/ou fraction de celui-ci.

Toutefois, lorsque le microorganisme annexe appartient à la même espèce que celle figurant le lysat requis selon l'invention, il est, de préférence, mis en oeuvre sous une forme différente d'un lysat.

Par contre, lorsque le microorganisme annexe appartient à une espèce différente que celle figurant le lysat requis selon la présente invention, il est également mis en oeuvre sous forme de lysat.

Au sens de la présente invention, on entend par « microorganisme probiotique », un microorganisme vivant qui, lorsqu'il est consommé en quantité adéquate, a un effet positif sur la santé de son hôte « joint FAO/WHO Expert Consultation on Evaluation of Health and Nutritional Properties of Probiotic in Food Including Powder Milk with Live Lactic Acid Bacteria, 6 octobre 2001 », et qui peut en particulier améliorer l'équilibre microbien intestinal.

Ces microorganismes convenant à l'invention peuvent être choisis notamment parmi les ascomycetes telles que *Saccharomyces, Yarrowia, Kluyveromyces, Torulaspora, Schizosaccharomyces pombe, Debaromyces, Candida, Pichia, Aspergillus* et *Penicillium,* des bactéries du genre *Bacteroides, Fusobacterium, Melissococcus, Propionibacterium, Enterococcus, Lactococcus, Staphylococcus, Peptostrepococcus, Bacillus, Pediococcus, Micrococcus, Leuconostoc, Weissella, Aerococcus, Oenococcus et Lactobacillus* et leurs mélanges.

Comme ascomycètes convenant tout particulièrement à la présente invention, on peut en particulier citer *Yarrowia lipolitica et Kluyveromyces lactis,* de même que *Saccharomyces cereviseae, Torulaspora, Schizosaccharamyces pombe, Candida* et *Pichia.*

Des *exemples spécifiques de microorganismes probiotiques sont Lactobacillus acidophilus, Lactobacillus alimentarius, Lactobacillus curvatus, Lactobacillus delbruckii subsp. Lactis, Lactobacillus gasseri, Lactobacillus johnsonii, Lactobacillus reuteri, Lactobacillus paracasei, Lactobacillus rhamnosus (Lactobacillus GG), Lactobacillus sake, Lactococcus lactis, Streptococcus thermophilus, Staphylococccus carnosus, et Staphylococcus xylosus* et leurs *mélanges.*

Plus particulièrement, il s'agit de microorganismes probiotiques issus du groupe des bactéries lactiques, comme notamment les *Lactobacillus.* A titre illustratif de ces bactéries lactiques, on peut plus particulièrement citer les *Lactobacillus johnsonii, Lactobacillus reuteri, Lactobacillus rhamnosus* et leurs mélanges.

Les espèces convenant tout particulièrement sont les *Lactobacillus johnsonii,* dont en particulier la souche déposée suivant le traité de Budapest avec l'Institut Pasteur (28 rue du Docteur Roux, F-75024 Paris cedex 15) sous la désignation suivante CNCM I-1225.

D'une manière générale, les compositions à application topique selon l'invention comprennent généralement de 0,0001 à 30 %, en particulier de 0,001 à 15 % et plus particulièrement de 0,1 à 10 % en poids en un ou plusieurs microorganismes notamment probiotiques, annexes.

Généralement, les compositions à application topique selon l'invention comprennent de 0,05 à 20 %, en particulier de 0,5 à 15 % et plus particulièrement de 0,5 à 10% en poids en lysat d'au moins un microorganisme, notamment probiotique, annexe.

Ce ou ces microorganisme(s) peu(ven)t donc être inclus dans les compositions selon l'invention sous une forme vivante, semi-active ou inactivée, morte.

Dans le cas où ces microorganismes sont formulés dans une composition sous une forme vivante, la quantité de microorganismes vivants peut varier de 10³ à 10¹⁵ ufc/g, en particulier de 10⁵ à 10¹⁵ ufc/g et plus particulièrement de 10⁷ à 10¹² ufc/g de microorganismes par gramme de composition.

Dans le cas particulier hors invention où le(s) microorganisme(s) est (sont) formulé(s) dans des compositions à administrer par voie orale, la concentration en microorganisme(s) notamment probiotique(s) peut être ajustée de manière à correspondre à des doses (exprimées en équivalent de microorganisme) variant de 5.10⁵ à 10¹³ ufc/j et en particulier de 10⁷ à 10¹¹ ufc/j.

Dans le cas particulier hors invention où le lysat de microorganisme(s) est formulé dans des compositions à administrer par voie orale, la concentration initiale en microorganisme(s), notamment probiotique(s), peut être ajustée de manière à correspondre à des doses (exprimées en équivalent de microorganisme) variant de 5.10⁵ à 10¹³ ufc/j et en particulier de 10⁷ à 10¹¹ ufc/j.

Il(s) peu(ven)t également être inclus sous forme de fractions de composants cellulaires. Le ou le(s) microorganisme(s), ou fraction(s) peu(ven)t également être introduit(s) sous la forme d'une poudre, d'un liquide, d'un surnageant de culture ou l'une de ses fractions, dilué(e) ou non, ou encore concentré(e) ou non.

Selon une variante, les compositions peuvent également contenir un cation minéral divalent.

Les compositions selon l'invention peuvent se présenter sous toutes les formes galéniques normalement disponibles pour le mode d'administration retenu.

Le support peut être de nature diverse selon le type de composition considérée.

En ce qui concerne plus particulièrement les compositions destinées à une administration par voie topique externe c'est-à-dire en surface de la peau, il peut s'agir de solutions aqueuses, hydroalcooliques ou huileuses, de dispersions du type des solutions ou dispersions du type lotion ou sérum, d'émulsions de consistance liquide ou semi-liquide du type lait, de suspensions ou émulsions, du type crème, de gel aqueux ou anhydre, de microémulsions, de microcapsules, de microparticules, ou de dispersions vésiculaires de type ionique et/ou non ionique.

Ces compositions sont préparées selon les méthodes usuelles.

Ces compositions peuvent notamment constituer des crèmes de nettoyage, de protection, de traitement ou de soin pour le visage, pour les mains, pour les pieds, pour les grands plis anatomiques ou pour le corps, (par exemple crèmes de jour, crèmes de nuit, crèmes démaquillantes, crèmes de fond de teint, crèmes anti-solaires), des produits de maquillage comme des fonds de teint fluides, des laits de démaquillage, des laits corporels de protection ou de soin, des laits après-solaires, des lotions, gels ou mousses pour le soin de la peau, comme des lotions de nettoyage ou de désinfection, des lotions anti-solaires, des lotions de bronzage artificiel, des compositions pour le bain, des compositions déodorantes contenant un agent bactéricide, des gels ou lotions après-rasage, des crèmes épilatoires, ou des compositions contre les piqûres d'insectes.

Les compositions selon l'invention peuvent également consister en des préparations solides constituant des savons ou des pains de nettoyage.

Elles peuvent être également utilisées pour le cuir chevelu sous forme de solutions, de crèmes, de gels, d'émulsions, de mousses ou encore sous forme de compositions pour aérosol contenant également un agent propulseur sous pression.

Lorsque la composition de l'invention est une émulsion, la proportion de la phase grasse peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine cosmétique et/ou dermatologique. L'émulsionnant et le coémulsionnant peuvent être présents, dans la composition, en une proportion allant de 0,3 à 30 % en poids, et de préférence de 0,5 à 20 % en poids par rapport au poids total de la composition.

Lorsque la composition de l'invention est une solution ou un gel huileux, la phase grasse peut représenter plus de 90 % du poids total de la composition.

De façon connue, les formes galéniques dédiées à une administration topique peuvent contenir également des adjuvants habituels dans le domaine cosmétique, pharmaceutique et/ou dermatologique, tels que les gélifiants hydrophiles ou lipophiles, les actifs hydrophiles ou lipophiles, les conservateurs, les antioxydants, les solvants, les parfums, les charges, les filtres, les bactéricides, les absorbeurs d'odeur et les matières colorantes. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse et/ou dans la phase aqueuse.

Comme matières grasses utilisables dans l'invention, on peut citer les huiles minérales comme par exemple le polyisobutène hydrogéné et l'huile de vaseline, les huiles végétales comme par exemple une fraction liquide du beurre de karité, huile de tournesol et d'amandes d'abricot, les huiles animales comme par exemple le perhydrosqualène, les huiles de synthèse notamment l'huile de Purcellin, le myristate d'isopropyle et le palmitate d'éthyl hexyle, les acides gras insaturés et les huiles fluorées comme par exemple les perfluoropolyéthers. On peut aussi utiliser des alcools gras, des acides gras comme par exemple l'acide stéarique et comme par exemple des cires notamment de paraffine, carnauba et la cire d'abeilles. On peut aussi utiliser des composés siliconés comme les huiles siliconées et par exemple les cyclométhicone et diméthicone, les cires, les résines et les gommes siliconées.

Comme émulsionnants utilisables dans l'invention, on peut citer par exemple le stéarate de glycérol, le polysorbate 60, le mélange alcool cétylstéarylique/alcool cétylstéarylique oxyéthyléné à 33 moles d'oxyde d'éthylène vendu sous la dénomination Sinnowax AO^{®} par la société HENKEL, le mélange de PEG-6/PEG-32/Glycol Stéarate vendu sous la dénomination de Tefose^{®} 63 par société GATTEFOSSE, le PPG-3 myristyl éther, les émulsionnants siliconés tels que le cétyldiméthicone copolyol et le mono- ou tristéarate de sorbitane, le stéarate de PEG-40, le monostéarate de sorbitane oxyéthyléné (20OE).

Comme solvants utilisables dans l'invention, on peut citer les alcools inférieurs, notamment l'éthanol et l'isopropanol, le propylène glycol.

La composition de l'invention peut également contenir de façon avantageuse une eau thermale et/ou minérale, notamment choisie parmi l'eau de Vittel, les eaux du bassin de Vichy et l'eau de la Roche Posay.

Comme gélifiants hydrophiles, on peut citer les polymères carboxyliques tel que le carbomer, les copolymères acryliques tels que les copolymères d'acrylates/alkylacrylates, les polyacrylamides et notamment le mélange de polyacrylamide, C13-14-Isoparaffine et Laureth-7 vendu sous le nom de Sepigel 305^{®} par la société SEPPIC, les polysaccharides comme les dérivés cellulosiques tels que les hydroxyalkylcelluloses et en particulier les hydroxypropylcellulose et hydroxyéthylcellulose, les gommes naturelles telles que les guar, caroube et xanthane et les argiles.

Comme gélifiants lipophiles, on peut citer les argiles modifiées comme les bentones, les sels métalliques d'acides gras comme les stéarates d'aluminium et la silice hydrophobe, ou encore l'éthylcellulose et le polyéthylène.

Dans le cas d'une utilisation d'une administration par voie orale, hors invention et uniquement décrite dans le présent texte, on considère l'utilisation d'un support ingérable.

Conviennent notamment comme supports alimentaires ou pharmaceutiques, le lait, le yaourt, le fromage, les laits fermentés, les produits fermentés à base de lait, des glaces, des produits à base de céréales fermentées, des poudres à base de lait, des formules pour enfants et nourrissons, des produits alimentaires de type confiserie, chocolat, céréales, des aliments pour animaux en particulier domestiques, des comprimés, gélules ou tablettes, des suppléments oraux sous forme sèche et les suppléments oraux sous forme liquide.

Pour l'ingestion, de nombreuses formes de réalisation de compositions orales, hors invention et uniquement décrite dans le présent texte, et notamment de compléments alimentaires sont possibles. Leur formulation est réalisée par les procédés usuels pour produire des dragées, gélules, gels, émulsions, comprimés, capsules. En particulier, le(s) actif(s) selon l'invention peuvent être incorporés dans toute autre forme de compléments alimentaires ou d'aliments enrichis, par exemple des barres alimentaires, ou des poudres compactées ou non. Les poudres peuvent être diluées à l'eau, dans du soda, des produits laitiers ou dérivés du soja, ou être incorporées dans des barres alimentaires.

Selon un mode de réalisation particulier, les microorganismes annexés peuvent être formulés au sein de compositions sous une forme encapsulée de manière à améliorer significativement leur durée de survie. Dans un tel cas, la présence d'une capsule peut en particulier retarder ou éviter la dégradation du microorganisme au niveau du tractus gastro intestinal.

Bien entendu, les compositions topiques externes selon invention, ou orales hors invention, ou associations selon l'invention peuvent en outre contenir plusieurs autres actifs.

A titre d'actifs conventionnellement mis en oeuvre, on peut citer, les vitamines B3, B5, B6, B8, C, E, ou PP, la niacine, les caroténoïdes, les polyphénols et minéraux tels que zinc, calcium, magnésium ....

En particulier, on peut utiliser un complexe anti-oxydant comprenant les vitamines C et E, et au moins un caroténoïde, notamment un caroténoïde choisi parmi le β-carotène, le lycopène, l'astaxanthine, la zéaxanthine et la lutéine, des flavonoïdes telles que les catéchines, l'hespéridine, des proanthocyanidines et des anthocyanines.

Il peut également s'agir d'au moins un prébiotique ou un mélange de prébiotiques. Plus particulièrement, ces prébiotiques peuvent être choisis parmi les oligosaccharides, produits à partir du glucose, galactose, xylose, maltose, sucrose, lactose, amidon, xylane, l'hémicellulose, l'inuline, des gommes de type acacia par exemple, ou un de leurs mélanges. Plus particulièrement, l'oligosaccharide comprend au moins un fructo-oligosaccharide. Plus particulièrement, ce prébiotique peut comprendre un mélange de fructo-oligosaccharide et d'inuline.

Dans les formes galéniques topiques, on peut utiliser plus particulièrement comme actifs hydrophiles les protéines ou les hydrolysats de protéine, les acides aminés, les polyols notamment en C₂ à C₁₀ comme les glycérine, sorbitol, butylène glycol et polyéthylène glycol, l'urée, l'allantoïne, les sucres et les dérivés de sucre, les vitamines hydrosolubles, l'amidon, des extraits bactériens ou végétaux comme ceux d'Aloe Vera.

Pour ce qui est des actifs lipophiles, on peut utiliser le rétinol (vitamine A) et ses dérivés, le tocophérol (vitamine E) et ses dérivés, les céramides, les huiles essentielles et les insaponifiables (tocotriènol, sésamine, gamma oryzanol, phytostérols, squalènes, cires, terpènes).

Le lysat est mis en oeuvre dans une composition topique destinée à la peau avec au moins un agent à effet secondaire irritant.

En effet, des composés topiques dont l'utilisation peut, dans des circonstances particulières telles qu'une peau réactive des concentrations élevées desdits composés..., conduire à l'apparition de sensations d'inconfort et/ou signes cutanés, sont utilisés dans des compositions cosmétiques ou dermatologiques, bien entendu pour d'autres effets.

En outre, même certains composés qui sont considérés comme inertes dans une composition cosmétique ou dermatologique, tels que, par exemple, les conservateurs, les tensioactifs, les parfums, les solvants ou les propulseurs, peuvent présenter un caractère irritant lorsqu'ils sont appliqués sur les matières kératiniques et notamment la peau y compris le cuir chevelu, ce caractère irritant dépendant du composé utilisé et de la sensibilité de la peau et de la flore cutanée résidente de l'utilisateur.

Plus particulièrement à titre d'actifs dermatologiques ou cosmétiques susceptibles d'avoir un effet secondaire irritant, on peut citer certains agents desquamants.

Parmi ces agents desquamants les suivants sont susceptibles de provoquer une irritation de la peau : les acides monocarboxyliques saturés (acide acétique) et insaturés, les acides dicarboxyliques saturés et insaturés, les acides tricarboxyliques saturés et insaturés ; les α-hydroxyacides et β-hydroxyacides des acides monocarboxyliques ; les α-hydroxyacides et β-hydroxyacides des acides dicarboxyliques ; les α-hydroxyacides et β-hydroxyacides, des acides tricarboxiliques, les cétoacides, les α-cétoacides, les β-cétoacides d'acides polycarboxyliques, d'acides polyhydroxy monocarboxyliques, d'acides polyhydroxy bicarboxyliques et d'acides polyhydroxy tricarboxyliques.

Particulièrement parmi les α-hydroxyacides ou leurs esters on peut citer : les acides glycolique, dioïque comme l'acide octadécène dioïque ou Arlatone dioc DCA vendu par la société Uniqema, citrique, lactique, tartrique, malique ou mandélique, leur esters comme le tartrate de dialkyle (C12/C13) ou Cosmacol ETI, le citrate de tri-alcools C12-13 ramifiés ou Cosmaco1 ECI commercialisé par la société SASOL.

Parmi les β-hydroxyacides on peut citer : l'acide salicylique et ses dérivés (dont l'acide n-octanoyl 5-salicylique).

Parmi les autres agents desquamants on peut citer : les acides pyruvique, gluconique, glucuronique, oxalique, malonique, succinique, acétique, gentisique, cinnamique, azélaique ; le phénol ; la résorcine ; l'urée et ses dérivés, l'hydroxyéthyl urée ou hydrovance^{®} de chez NATIONAL STARCH ; les oligofucoses ; l'acide jasmonique et ses dérivés ; l'acide trichloracétique ; l'extrait de Saphora japonica et le résvératrol.

Parmi les agents desquamants, ceux capables d'agir sur les enzymes impliqués dans la desquamation ou la dégradation des cornéodesmosomes peuvent également être susceptibles de provoquer une irritation de la peau.

Parmi ceux-ci, on peut notamment citer les agents chélatants des sels minéraux tels l'EDTA ; l'acide N-acyl-N,N',N'éthylène diaminetriacétique ; les composés aminosulfoniques et en particulier l'acide (N-2 hydroxyéthylpiperazine-N-2-éthane) sulfonique (HEPES) ; les dérivés de l'acide 2-oxothiazolidine-4-carboxylique (procystéine) ; les dérivés d'acides alpha aminés de type glycine (tels que décrits dans EP 0 852 949, ainsi que le méthyl glycine diacétate de sodium commercialisé par BASF sous la dénomination commerciale TRILON M^{®}) ; le miel ; les dérivés de sucre tels que l'O-octanoyl-6-D-maltose, le O-linoleyl-6-D-glucose et la N-acétyl glucosamine. (à conserver ?)oui à conserver

Les rétinoïdes sont également des composés susceptibles de provoquer une irritation de la peau. On peut par exemple citer parmi eux le rétinol et ses esters, le rétinal, l'acide rétinoïque et ses dérivés tels que ceux décrits dans les documents FR-A-2 570 377, EP-A-199 636, EP-A-325 540, EP-A-402 072, et l'adapalène.

Les sels et dérivés, comme les formes cis ou trans, les mélanges racémiques, les formes dextrogyres ou levogyres des composés cités précédemment sont aussi considérés comme des composés susceptibles de provoquer une irritation de la peau.

D'autres actifs dermatologiques ou cosmétiques susceptibles de provoquer en effet secondaire une irritation de la peau sont également cités ci-après :
- l'urée et ces dérivés comme l'hydroxyéthyl urée ou hydrovance^{®} de NATIONAL STARCH,
- la vitamine D et ses dérivés tels que la vitamine D3, la vitamine D2, le calcitriol, le calcipotriol, le tacalcitol, la 24,25-diOH vitamine D3, la 1-OH vitamine D2 et la 1,24-diOH vitamine D2 ; la vitamine B9 et ses dérivés,
- les peroxydes comme le peroxyde de benzoyle, l'eau oxygénée,
- les antichutes tels que le minoxidil et ses derivés tel que l'aminexyl,
- les teintures et les colorants capillaires comme les aminophénols et leurs dérivés tels que la para-phénylène diamine (p-PDA), la N-phenyl p-PDA, le toluène 2,5-diamine sulfate, la méta-phénylène diamine (m-PDA), la toluène 3,4-diamine et l'ortho-phénylène diamine (o-PDA),
- les agents anti-transpirants comme les sels d'Aluminium, tel que l'hydroxychlorure d'aluminium,
- les actifs de permanentes tels que les thioglycolates, l'ammoniaque,
- le thioglycolate et ses sels,
- le phénoxyéthanol,
- le 1,2-pentanediol,
- les solutions alcooliques parfumantes (parfums, eaux de toilette, après rasage, déodorants)
- les anthralines (dioxyanthranol),
- les anthranoïdes (par exemple ceux décrits dans le document EP-A- 319028),
- les sels de lithium,
- les dépigmentants (ex : hydroquinone, vitamine C à forte concentration, acide kojique),
- certains actifs amincissants à effet chauffant,
- les nicotinates et leurs dérivés,
- la capsaïcine,
- les actifs antipoux (pyréthrine),
- les antiprolifératifs tels que le 5-fluoro uracile ou le méthotrexate,
- les agents antiviraux,
- les antiparasitaires,
- les antifongiques,
- les antiprurigineux,
- les antiséborrhéiques,
- certaines filtres solaires,
- les propigmentants tels que les psoralènes et les méthylangécilines, et
- leurs mélanges.

Selon un mode de réalisation préféré de l'invention, le composé susceptible de provoquer en effet secondaire une irritation de la peau est choisi parmi les rétinoïdes, les α-hydroxyacides, les β-hydroxyacides, les acides dicarboxyliques saturés et insaturés tels que l'acide octadécène dioique ou Arlatone DIOC DCA vendu par la société Uniqema, les tensioactifs anioniques, cationiques ou amphotères, l'acide n-octanoyl 5-salicylique, les actifs antiperspirants tels que les sels d'aluminium, l'acide (N-2 hydroxyéthylpiperazine-N-2-éthane) sulfonique (HEPES) et l'acide cinnamique.

Le composé susceptible de provoquer en effet secondaire une irritation de la peau, peut être présent dans la composition selon la présente invention dans une quantité suffisante pour provoquer une réaction d'irritation de la peau. A titre d'exemple, il peut être présent dans une teneur allant de 0,0001 à 70 % en poids, de préférence de 0,01 à 50 % en poids et mieux de 0,1 à 30 % en poids par rapport au poids total de la composition.

Comme autres actifs susceptibles d'être plus particulièrement associés au lysat dans une formule galénique orale décrite dans le présent texte mais hors invention, on peut également considérer tous les ingrédients communément utilisés et/ou autorisés.

A titre illustratif, on peut citer les vitamines, les minéraux, les lipides essentiels, les oligoéléments, les polyphénols, les flavonoïdes, les phytoestrogènes, les antioxydants tels que l'acide lipoïque et le coenzyme Q10, les caroténoïdes, les probiotiques, les prébiotiques, les protéines et les acides aminés, les mono et polysaccharides, les amino-sucres, les phytostérols et alcools triterpéniques d'origine végétale.

Il s'agit, en particulier, des vitamines A, C, D, E, PP et du groupe B. Parmi les caroténoïdes, on choisit de préférence, le béta-carotène, le lycopène, la lutéine, la zéazanthine et l'astaxanthine. Les minéraux et oligo-éléments particulièrement mis en oeuvre sont le zinc, le calcium, le magnésium, le cuivre, le fer, l'iode, le manganèse, le sélénium, le chrome (III). Parmi les polyphénols, on retient aussi en particulier les polyphénols de raisin, de thé, d'olive, de cacao, de café, de pomme, de myrtille, de sureau, de fraise, de canneberge, et d'oignon. De préférence parmi les phytoestrogènes, on retient les isoflavones sous la forme libre ou glycosylée, telles que la génistéine, la daidzéine, la glycitéine ou encore les lignanes, en particulier ceux du lin et du schizandra chinensis. Les probiotiques sont choisis de préférence dans le groupe constitué par les lactobacilles et les bifidobactéries. Les acides aminés ou les peptides et les protéines les contenant, tels que la taurine, la thréonine, la cystéine, le tryptophane, la méthionine. Les lipides appartiennent de préférence au groupe des huiles contenant des acides gras mono et polyinsaturés tels que les acides oléique, linoléique, alpha-linolénique, gamma-linolénique, stearidonique, les acides gras oméga-3 de poisson à longue chaîne tels que l'EPA et le DHA, les acides gras conjugués issus de végétaux ou d'animaux tels que les CLA (Conjugated Linoleic Acid).

Le procédé de traitement cosmétique peut être mis en oeuvre notamment en administrant les compositions cosmétiques et/ou dermatologiques ou associations telles que définies ci-dessus, selon la technique d'utilisation habituelle de ces compositions. Par exemple : applications de crèmes, de gels, de sérums, de lotions, de laits de démaquillage ou de compositions après-solaires sur la peau ou sur les cheveux secs, application d'une lotion pour cheveux sur cheveux mouillés ou de shampooings pour ce qui est de l'application topique.

Le procédé cosmétique est ainsi mis en oeuvre par administration topique, journalière par exemple, du lysat considéré selon l'invention.

Le procédé peut comprendre une administration unique. Selon un autre mode de réalisation, l'administration est répétée par exemple 2 à 3 fois quotidiennement sur une journée ou plus et généralement sur une durée prolongée d'au moins 4 semaines, voire 4 à 15 semaines, avec le cas échéant une ou plusieurs périodes d'interruption.

L'utilisation conforme à l'invention est telle que les lysats ou compositions définis ci-dessus sont mis en oeuvre dans une formulation destinée à un usage topique.

Dans la description et dans les exemples suivants, sauf indication contraire, les pourcentages sont des pourcentages en poids et les plages de valeurs libellées sous la forme « entre ... et ... » incluent les bornes inférieure et supérieure précisées. Les ingrédients sont mélangés, avant leur mise en forme, dans l'ordre et dans des conditions facilement déterminées par l'homme de l'art.

Les exemples ci-après sont présentés à titre illustratif et non limitatif du domaine de l'invention.

### Exemple 1 :

**Lotion pour le visage des peaux sensibles**

| | |
|---|---|
| *Lysat de Bifidobacterium longum** | 5,00** |
| Gluconate de magnésium | 3,00 |
| Lactate de calcium | 2,00 |
| Antioxydant | 0,05 |
| Isopropanol | 40,0 |
| Conservateur | 0,30 |
| Eau | qsp 100 % |
| *Repair Complex CLR^{®} commercialisé par K.Richter GmbH et correspondant à une formulation à 5 % en poids d'actifs | |
| ** quantité exprimée en produit total | |

### Exemple 2 :

**Gel pour le soin du visage des peaux sensibles**

| | |
|---|---|
| Nitrate de strontium | 4,00 |
| Lysat de *Bifïdobacterium longum* * | 5,00** |
| Hydroxypropylcellulose (Klucel H^{®} vendu par la société HERCULES) | 1,00 |
| Vitamine E | 2,50 |
| Antioxydant | 0,05 |
| Isopropanol | 40,00 |
| Conservateur | 0,30 |
| Eau | qsp 100 % |
| * Repair Complex CLR^{®} commercialisé par K.Richter GmbH et correspondant à une formulation à 5 % en poids d'actifs | |
| ** quantité exprimée en produit total | |

### Exemple 3 :

**Crème pour le soin des peaux sensibles**

| | |
|---|---|
| Hydroxyde de sodium | 0,07 |
| Disodium EDTA | 0,05 |
| Glycine | 1 |
| Lysat de *Bifidobacterium longum** | 5,00** |
| Phenoxyéthanol | 0,80 |
| Benzoate de sodium | 0,30 |
| Paraffinum liquidum | 8 |
| Butyrospermum parkii | 20 |
| Alcool cétylique | 0,50 |
| Huile de Canola | 5 |
| VP/copolymère d'eicosène | 1 |
| Carbomer^{®} | 0,25 |
| Cyclopentasiloxane | 5 |
| Cyclopentasiloxane et dimethiconol | 3 |
| Glycérine | 10 |
| Pentylène glycol | 1 |
| Acide stéarique | 1,25 |
| Polysorbate 61 | 1,50 |
| Glutamate de disodium stearoyl | 1 |
| Tristéarate de saccharose | 2,25 |
| Tocophérol | 0,30 |
| Eau | qsp 100 % |
| * Repair Complex CLR^{®} commercialisé par K.Richter GmbH et correspondant à une formulation à 5 % en poids d'actifs | |
| ** quantité exprimée en produit total | |

### Exemple 4

### Evaluation de l'efficacité d'un lysat de Bifidobacterium longum vis-à-vis de la réactivité d'une peau sensible.

Le produit testé est un lysat de *Bifidobacterium longum* en suspension désintégrée (aux ultrasons) dans un milieu aqueux faiblement acide commercialisé sous le nom Repair Complex CLR^{®}. L'actif est à 5 % en poids dans ce biolysat total commercialisé.

L'actif a été testé seul dans une étude randomisée en double aveugle.

Soixante six femmes présentant des peaux sèches ont été réparties en deux groupes, placebo (n=33) (groupe A), Repair Complex CLR^{®} (n=33) (groupe B). Les traitements ont été appliqués topiquement durant 58 jours, l'actif étant formulé à 10 % de la formulation testée. Cette formule support est une émulsion H/E déminéralisée Arlacel/myrj^{®} contenant 5 % Parleam, 15 % de cyclopentasiloxane, 3% glycérine et 2 % vaseline.
Dans la formule placebo, l'absence de Repair Complex CLR^{®} est compensée par de l'eau.

Afin de suivre l'évolution de la sensibilité cutanée, un stinging test a été réalisé à l'inclusion puis régulièrement tout au long de l'étude.

Le test utilisé est le Lactic Acid Stinging test ou "test du picotement", mis au point par Frosch et Kligman en 1977 (GREEN BG, BLUTH J. Measuring the chemosensory irritability of human skin. J Toxicol - Cut & Ocular Toxicol ; 14(1), 23-48 (1995). Il permet d'évaluer la réactivité cutanée.

Ce test détermine la capacité des individus en général, à ressentir des picotements après application d'une solution d'acide lactique au niveau des sillons naso-géniens. Ces régions sont en effet très réactives et leur couche cornée très perméable. Elles sont riches en follicules pileux et en glandes sudoripares, ce qui favorise la pénétration des produits. Enfin, elles disposent d'un réseau nerveux sensoriel périphérique très dense.

Le test décrit ci-dessus repose sur la cotation par le sujet, au cours du temps, du degré des sensations d'inconfort induites par l'application topique, sur une zone du visage, d'un actif stimulant les nerfs sensitifs cutanés. La pertinence de ce test a été montrée puisque, globalement, les sujets à peau sensible rapportent un inconfort plus important.

Dans ce test, l'agent stimulant la réactivité cutanée est administré topiquement à une dose unique et identique pour tous les sujets (10% d'acide lactique). La cotation de l'inconfort est faite sur une échelle du type 0 = pas de sensation, 1 = sensation légère, 2 = sensation modérée et 3 = sensation forte.

Les sujets ont été évalués à J1, J29, J43, et J57. A chaque visite les sujets ont évalués la sensation de picotement à 30 secondes, 2 minutes, et 5 minutes après application d'acide lactique et de sérum physiologique, selon une échelle allant de 0 pour pas de picotement à 4 pour une sensation de sévère picotement. Le score global de réactivité est calculé en faisant la différence de la somme des scores côté acide lactique moins la somme des scores côté sérum physiologique.

Le tableau I ci-dessous représente par visite et par traitement les moyennes et intervalles de confiance à 95% de la moyenne pour le score de stinging.

**TABLEAU I**

| **Groupe** | **Visite** | **N** | **Moyenne** | **Erreur standard de la moyenne** |
|---|---|---|---|---|
| A | 1 | 32 | 4,88 | ,361 |
| | 29 | 30 | 3,60 | ,409 |
| | 43 | 29 | 2,62 | ,448 |
| | 57 | 28 | 4,04 | ,453 |
| B | 1 | 31 | 5,29 | ,360 |
| | 29 | 30 | 3,40 | ,370 |
| | 43 | 30 | 2,40 | ,409 |
| | 57 | 30 | 2,27 | ,262 |

La diminution générale du score de stinging observée sur le tableau 1 ressort très significative au cours du temps (facteur visite, test de Fisher, p<0.0001). De plus, il existe une interaction entre les traitements et le jour de visite (test de Fisher, p=0.019).

A J57, on note une nette augmentation du score de stinging dans le groupe A, alors qu'il semble stabilisé dans le groupe B. Cela se traduit par une augmentation de la sensibilité dans le groupe placebo à J57 alors qu'elle reste diminuée dans le groupe B actif jusqu'au J57.

Le produit à l'essai induit une amélioration significative contre placebo de la sensibilité cutanée après deux mois de traitement.

L'évaluation du seuil de sensibilité par le stinging test montre que la formule à 10% de Repair Complex CLR^{®} diminue significativement la sensibilité cutanée des volontaires à peau sensible (p=0,0024) à J57 contre placebo, après application topique d'une formule à 10%.

### Exemple 5

Evaluation de l'effet apaisant d'un lysat conforme à la présente invention

### - Cellules utilisées

Primo culture de neurones sensitifs préparés selon la technique décrite par Hall et collaborateurs (J Neurosci. 1997 ;17(8):2775-84)

### - Milieu de culture:

DMEM-HAM F12 (Invitrogen 21331-020)
L-glutamine 2mM (Invitrogen 25030024)
Pénicilline 50 UI/ml - Streptomycine 50 µg/ml (Invitrogen 15070063)
Supplément N2 (17502-048)
Nerf Growth Factor (NGF, Invitrogen 13290.010)
Neurotrophine 3 (NT-3, Tebu 450-03-b)

### Produits testés

| | Dilution | Concentrations finales testées |
|---|---|---|
| Repair Complex CLR^{®} Lysat selon l'invention | en milieu de culture | 3%, 1% et 0.1% |
| Capsaïcine (Sigma M2028) 10⁻² M dans éthanol | en milieu de culture | 10⁻⁶ M |

### - Culture de neurones sensitifs

Les neurones sensitifs ont été cultivés en plaque 96 puits (30 000 cellules par puits) en milieu défini dans une étuve à 37°C et 5 % CO₂ saturée en humidité. Le milieu défini favorise le développement des neurones au détriment des cellules contaminantes (cellules de Schwann et fibroblastes). Après 9 jours de culture, les neurones électro-physiologiquement matures peuvent libérer spontanément des neuropeptides.

### - Traitement et dosage du CGRP

Après 10 jours de culture, les neurones ont été incubés en présence du composé « Repair Complex CLR^{®}» aux concentrations sélectionnées par le commanditaire.

Les neurones ont été incubés en présence du produit à tester pendant 6 heures et 20 minutes dans du milieu neurones sensitifs. Chaque condition expérimentale a été réalisée en quadruplicate.

A la fin de ces incubations, le milieu a été remplacé par du milieu DMEM contenant le produit à l'essai avec ou sans capsaïcine à 10⁻⁶M. Après 25 minutes d'incubation, les surnageants ont été recueillis et le contenu en CGRP a été mesuré dans les surnageants de culture par un test ELISA (Rat CGRP enzyme immunoassay Kit A05482).

### Résultats

Les résultats sont présentés dans le tableau II ci-après.

**TABLEAU II**

| **Libération de CGRP induite par la capsaïcine (10⁻⁶ M)-Incubation 6 heures** | | | | | | | |
|---|---|---|---|---|---|---|---|
| Condition de culture | quantité. | **CGRP libérée (pg/ml)** | | | | | |
| | | **Valeurs** | **Moyenne** | **Ecartype** | **n** | **% témoin** | ***p*** |
| | | 113.7 | | | | | |
| **Milieu témoin** | - | 80.7 | **91.0** | **16.4** | **4** | **100** | - |
| | | 77.3 | | | | | |
| | | 92.3 | | | | | |
| **Repair Complex CLR^{®}** | | 34.8 | | | | | |
| | **1 %** | 37.1 | **32.6** | **5.3** | **4** | **36** | **p < 0.01** |
| | | 33.3 | | | | | |
| | | 25.0 | | | | | |
| | | 54.6 | | | | | |
| | **0.30 %** | 27.3 | **37.5** | **12.2** | **4** | **41** | **p < 0.01** |
| | | 37.9 | | | | | |
| | | 30.3 | | | | | |

On note qu'après irritation des neurones par de la capsaïcine (10⁻⁶ M), le lysat testé à 1% et 0.3% a provoqué une diminution significative de la libération de CGRP (respectivement 36% et 41% du témoin, p<0.01).

Ces résultats témoignent des propriétés apaisantes en inhibant l'activité des neurones sensitifs après une irritation.

### Exemple 6

### Evaluation de l'activité d'un lysat selon l'invention sur une réaction neurogène

Pour cet essai, un modèle de peau humaine maintenue en survie stimulée par un neuromédiateur (la substance P « SP ») a été utilisé.

Ce neuromédiateur est en effet l'un des agents responsable de la réponse inflammatoire. A côté de ses effets vasculaires (oedème, vasodilatation, expression d'ELAM-1 sur la paroi des cellules endothéliales), SP induit également des réactions biochimiques avec formation de nitrites dans les cellules endothéliales et libération de médiateurs pro-inflammatoires (IL1a, IL6, TNFα).

L'évaluation a été réalisée histologiquement par analyse quantitative du nombre de mastocytes dégranulés et de la modulation vasculaire et biochimiquement par dosage de TNFα.

8 peaux humaines de donneurs différents ont été obtenues chez des femmes (âge entre 30 et 55 ans) après chirurgie plastique et maintenues en survie ex vivo .

Les fragments de peau sont déposés dans des inserts eux-mêmes disposés en suspension au-dessus de puits de culture. Du milieu (Milieu Essentiel Minimum de Dulbecco, D-MEM) (antibiotiques, SVF) a été ajouté dans le fond des puits, un passage s'effectuant par diffusion lente entre les deux compartiments par l'intermédiaire d'une membrane poreuse (0,45 mm). 5 heures de ré-équilibrage sont nécessaires avant de débuter le protocole.

Au terme des 5 heures de ré-équilibrage, le modèle expérimental d'inflammation neurogène est réalisé en ajoutant 10 µM de substance P (SP) dans le milieu de culture. Le lysat Repair Complex^{®} est appliqué simultanément en topique à la concentration de 10% (volume appliqué: 40 µl/cm²). Les fragments de peau sont alors maintenus en culture d'organes pendant 24 heures dans une étuve à atmosphère humide, à 37 °C et en présence de 5 % de CO₂. Le produit est réappliqué à J1.

Une étude comparative a été réalisée entre les 3 conditions suivantes:
- peau témoin (condition de base : peau non stimulée, non traitée)
- peau stimulée par la substance P 10 µM
- peau stimulée par SP et traitée par le lysat à 10%

Les protocoles d'analyses retenus sont les suivants :

### a) Evaluation histologique de l'oedème et des modifications du diamètre des capillaires

Les fragments de peaux ont été fixés dans le liquide de Bouin et inclus en paraffine. Après coloration par l'hémalun-éosine, 2 critères sont évalués au niveau du derme: le calibre des capillaires et l'oedème.

### b) Evaluation du calibre des capillaires

Après coloration par l'hémalun-éosine, la dilatation vasculaire a été évaluée par un comptage du nombre de vaisseaux dilatés sur l'ensemble de la coupe histologique (16 champs au grossissement 40). Ce nombre est rapporté au nombre total de vaisseaux afin de calculer le pourcentage de vaisseaux dilatés. Par ailleurs, une analyse morphométrique de la surface occupée par la lumière des vaisseaux a été réalisée afin de déterminer la surface moyenne (µm²) occupée par les vaisseaux.

### c) Evaluation histologique de l'oedème

L'évaluation de l'oedème est réalisée à l'aide de scores semi-quantitatifs:
- pas d'oedème: 0 (score 0)
- oedème très léger: + (score 1)
- oedème modéré: ++ (score 2)
- oedème important: +++ (score 3)
- oedème très important: ++++ (score 4)

### d) Dosage de cytokine pro-inflammatoire

La modulation de la sécrétion de cytokines telle que le TNF a été recherchée□.

Le dosage de cette cytokine est réalisé par une technique d'immuno-essai avec lecture spectrophotométrique de la concentration (pg/ml) (kits de dosage Chemicon International, INC). Les fragments de peau ayant la même surface (vérification du poids de chaque fragment), le dosage a été réalisé à partir des surnageants de culture.

### e) Analyses statistiques

Une moyenne a été réalisée à partir des résultats obtenus sur les 8 peaux. L'analyse statistique est effectuée par le test de Student dit de l'écart réduit ou test des échantillons appariés, avec un risque de 5%.

### Résultats

### a) Evaluation de la modulation vasculaire

### - Evaluation du % global de capillaires dilatés

Le Tableau III ci-après rend compte de ces résultats.

**Tableau III**

| | % |
|---|---|
| Peau témoin | 69 ± 18,9 |
| Peau + SP | 77,7 ± 17,8 |
| | p = 0,053 |
| Peau + SP + lysat | 69,06 ± 21,1 |
| | p = 0,054 |

Il est à noter que l'application de SP induit une vasodilatation par rapport à la peau témoin : 77,7 versus 69% (résultat proche de la significativité : p = 0,053).

L'application du lysat induit une diminution de ce % de vaisseaux dilatés : 69,06% par rapport au modèle de stimulation par SP.

### - Mesure de la surface moyenne des capillaires

Les résultats concernant la mesure de la surface moyenne des capillaires sont exposés dans le Tableau IV ci-après.

**Tableau IV**

| | | µm2 |
|---|---|---|
| Peau témoin | | 107,66 ± 71,63 |
| Peau + SP | | 144,4 ± 71,2 |
| | | * p = 0,02 |
| Peau + SP + lysat | | 97,4 ± 60,7 |
| | | # p = 0,0003 |
| | * : différence statistiquement significative par rapport à la peau témoin (test apparié de Student, p < 0,05) | |
| | # : différence statistiquement significative par rapport à la condition SP (test apparié de Student, p < 0,05) | |

Il est à noter que l'application de la SP induit une vasodilatation statistiquement significative par rapport à la peau témoin : 144,4 µm² versus 107,66 µm² (p = 0,02). L'application du lysat permet de diminuer très significativement la dilatation vasculaire : 97,4 µm² par rapport au modèle d'inflammation neurogène (p = 0,0003).

### b) Evaluation de l'oedème dermique

Les résultats concernant l'analyse de l'oedème dermique sont exposés dans le Tableau V.

**Tableau V**

| | score |
|---|---|
| Peau témoin | 1,1 ± 0,64 |
| Peau + SP | 1,8 ± 0,7 |
| | * p = 0,02 |
| Peau + SP + lysat | 1,17 ± 0,7 |
| | # p = 0,009 |
| * : différence statistiquement significative par rapport à la peau témoin (test apparié de Student, p < 0,05) | |
| # : différence statistiquement significative par rapport à la condition SP (test apparié de Student, p < 0,05) | |

L'application de la SP induit un oedème statistiquement significatif par rapport à la peau témoin : score de 1,8 versus 1,1 (p = 0,02). L'application du lysat permet de diminuer très significativement l'oedème dermique: le score observé est de 1,17 par rapport au modèle de stimulation par SP (p = 0,009).

### c) Dosage de TNF

Les résultats du dosage de TNF sont exposés dans le Tableau VI.

**Tableau VI**

| | Pg/ml |
|---|---|
| Peau témoin | 32,3 ± 23,8 |
| Peau + SP | 49,2 ± 24,9 |
| | * p = 0,00001 |
| Peau + SP + lysat | 28,8 ± 12,3 |
| | # p = 0,07 |
| * : différence statistiquement significative par rapport à la peau témoin (test apparié de Student, p < 0,05) | |
| # : différence statistiquement significative par rapport à la condition SP (test apparié de Student, p < 0,05) | |

L'application de SP induit une augmentation statistiquement significative du taux de TNF par rapport à la peau témoin : 49,2 pg/ml versus 32,3 pg/ml (p = 0,00001). L'application du lysat permet de diminuer significativement l'excrétion de cette cytokine pro-inflammatoire : 28,8 pg/ml (p = 0,007 par rapport au modèle de stimulation par SP).

### d) Conclusion

L'ensemble de ces résultats témoigne d'un effet apaisant du lysat avec diminution importante et statistiquement significative de la vasodilatation, et de l'oedème. La diminution significative de la libération de TNF est à rapprocher de ces résultats histologiques.

## Revendications

1. Utilisation cosmétique non-thérapeutique d'une quantité efficace d'au moins un lysat d'au moins un microorganisme du genre *Bifidobacterium* pour prévenir et/ou diminuer les sensations d'inconfort cutané au niveau d'une peau sensible, ledit lysat étant formé de tous les constituants biologiques intracellulaires et des constituants des parois et membranes cellulaires, lesdites sensations d'inconfort cutané étant choisies parmi les démangeaisons, les échauffements, les sensations de picotement et/ou de tiraillement,
ledit lysat étant administré par voie topique.

2. Utilisation selon la revendication précédente, dans laquelle ledit lysat de microorganisme est destiné à prévenir et/ou traiter les manifestations de sensations dysesthétiques au niveau des peaux sensibles.

3. Utilisation selon la revendication 1 ou 2, dans laquelle le microorganisme du genre *Bifidobacterium* est choisi parmi *Bifidobacterium longum Bifidobacterium bifidum, Bifidobacterium breve, Bifidobacterium animalis, Bifidobacterium lactis, Bifidobacterium infantis, Bifidobacterium adolescentis* ou *Bifidobacterium pseudocatenulatum* et leurs mélanges.

4. Utilisation selon l'une quelconque des revendications 1 à 3, dans laquelle le microorganisme du genre *Bifidobacterium* est le *Bifidobacterium longum.*

5. Utilisation selon l'une quelconque des revendications 1 à 4, dans laquelle ledit lysat comprend de 0,1 à 50 % en poids, en particulier de 1 à 20 % en poids de matière(s) active(s) dérivées(s) du microorganisme du genre *Bifidobacterium.*

6. Composition cosmétique et/ou dermatologique, utile pour prévenir et/ou traiter un trouble cutané au niveau d'une peau sensible par voie topique, comprenant, dans un milieu physiologiquement acceptable, au moins une quantité efficace d'au moins un lysat d'au moins un microorganisme du genre *Bifidobacterium*, ledit lysat étant formé de tous les constituants biologiques intracellulaires et des constituants des parois et membranes cellulaires, en association à au moins un composé susceptible de provoquer une irritation cutanée et comprenant en outre un microorganisme probiotique annexe et/ou un lysat d'au moins un microorganisme probiotique annexe.

7. Composition selon la revendication précédente, **caractérisée en ce que** ledit microorganisme annexe probiotique est choisi parmi les ascomycètes telles que *Saccharomyces, Yarrowia, Kluyveromyces, Torulaspora, Schizosaccharomyces pombe, Debaromyces, Candida, Pichia, Aspergillus* et *Pénicillium,* des bactéries du genre *Bifidobacterium, Bacteroides, Fusobacterium, Melissococcus, Propionibacterium, Enterococcus, Lactococcus, Staphylococcus, Peptostrepococcus, Bacillus, Pediococcus, Micrococcus, Leuconostoc, Weissella, Aerococcus, Oenococcus, Lactobacillus,* et leurs mélanges.

8. Composition selon l'une quelconque des revendications 6 ou 7, **caractérisée en ce que** le microorganisme annexe est issu du groupe des bactéries lactiques.

9. Composition selon l'une quelconque des revendications 6 à 8, **caractérisée en ce que** le microorganisme annexe probiotique et/ou une de ses fractions est présent à raison de 0,0001 à 30 %, en particulier de 0,001 à 15 % et plus particulièrement de 0,1 à 10 % en poids de ladite composition.

10. Composition selon l'une quelconque des revendications 6 à 9, **caractérisée en ce qu'**elle se présente sous forme de solutions aqueuses, hydroalcooliques ou huileuses, de dispersions du type des solutions ou dispersions du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-solide ou solide du type crème, de gel aqueux ou anhydre, ou encore de microémulsions, de microcapsules, de microparticules, ou de dispersions vésiculaires de type ionique et/ou non ionique.

## Patentansprüche

1. Nichttherapeutische kosmetische Verwendung einer wirksamen Menge mindestens eines Lysats von mindestens einem Mikroorganismus der Gattung *Bifidobacterium* zur Verhinderung und/oder Verringerung der Empfindungen von Hautunbehagen bei einer empfindlichen Haut, wobei das Lysat aus sämtlichen biologischen intrazellulären Bestandteilen und Bestandteilen der Zellwände und Zellmembranen gebildet ist, wobei die Empfindungen von Hautunbehagen aus Juckreiz, Wärmegefühl, Prickel- und/oder Ziehschmerzempfindungen ausgewählt sind,
wobei das Lysat auf topischem Wege verabreicht wird.

2. Verwendung nach dem vorhergehenden Anspruch, wobei das Mikroorganismus-Lysat dazu bestimmt ist, die Anzeichen von dysästhetischen Empfindungen bei empfindlicher Haut zu verhindern und/oder zu behandeln.

3. Verwendung nach Anspruch 1 oder 2, wobei der Mikroorganismus der Gattung *Bifidobacterium* ausgewählt ist aus *Bifidobacterium longum Bifidobacterium bifidum, Bifidobacterium breve, Bifidobacterium animalis, Bifidobacterium lactis, Bifidobacterium infantis, Bifidobacterium adolescentis* oder *Bifidobacterium pseudocatenulatum* und ihren Gemischen.

4. Verwendung nach einem der Ansprüche 1 bis 3, wobei der Mikroorganismus der Gattung *Bifidobacterium Bifidobacterium longum* ist.

5. Verwendung nach einem der Ansprüche 1 bis 4, wobei das Lysat von 0,1 bis 50 Gew.-%, insbesondere von 1 bis 20 Gew.-% Wirkstoff(e) aus Mikroorganismen der Gattung *Bifidobacterium* umfasst.

6. Kosmetische und/oder dermatologische Zusammensetzung, die zur Verhinderung und/oder Behandlung einer Hautstörung bei empfindlicher Haut auf topischem Wege geeignet ist, umfassend, in einem physiologisch verträglichen Medium, mindestens eine wirksame Menge mindestens eines Lysats von mindestens einem Mikroorganismus der Gattung *Bifidobacterium,* wobei das Lysat aus sämtlichen biologischen intrazellulären Bestandteilen und Bestandteilen der Zellwände und Zellmembranen gebildet ist, in Kombination mit mindestens einer Verbindung, die dazu geeignet ist, eine Hautreizung hervorzurufen, und weiterhin umfassend einen zusätzlichen probiotischen Mikroorganismus und/oder ein Lysat von mindestens einem zusätzlichen probiotischen Mikroorganismus.

7. Zusammensetzung nach dem vorhergehenden Anspruch, **dadurch gekennzeichnet, dass** der zusätzliche probiotische Mikroorganismus ausgewählt ist aus Ascomyceten, wie *Saccharomyces, Yarrowia, Kluyveromyces, Torulaspora, Schizosaccharomyces pombe, Debaromyces, Candida, Pichia, Aspergillus* und *Penicillium,* Bakterien der Gattung *Bifidobacterium, Bacteroides, Fusobacterium, Melissococcus, Propionibacterium, Enterococcus, Lactococcus, Staphylococcus, Peptostrepococcus, Bacillus, Pediococcus, Micrococcus, Leuconostoc, Weissella, Aerococcus, Oenococcus, Lactobacillus,* und ihren Gemischen.

8. Zusammensetzung nach einem der Ansprüche 6 oder 7, **dadurch gekennzeichnet, dass** der zusätzliche Mikroorganismus aus der Gruppe der Milchsäurebakterien stammt.

9. Zusammensetzung nach einem der Ansprüche 6 bis 8, **dadurch gekennzeichnet, dass** der zusätzliche probiotische Mikroorganismus und/oder eine seiner Fraktionen in einem Anteil von 0,0001 bis 30 %, insbesondere von 0,001 bis 15 % und noch spezieller von 0,1 bis 10 Gew.-% der Zusammensetzung vorhanden ist.

10. Zusammensetzung nach einem der Ansprüche 6 bis 9, **dadurch gekennzeichnet, dass** sie in Form von wässrigen, hydroalkoholischen oder öligen Lösungen, von Dispersionen vom Typ Lösungen oder Dispersionen vom Typ Lotion oder Serum, von Emulsionen flüssiger oder halbflüssiger Konsistenz vom Typ Milch, die durch Dispersion einer Fettphase in einer wässrigen Phase (O/W) oder umgekehrt (W/O) erhalten werden, oder von Suspensionen oder Emulsionen von weicher, halbfester oder fester Konsistenz vom Typ Creme, von wässrigem oder wasserfreiem Gel, oder auch von Mikroemulsionen, Mikrokapseln, Mikropartikeln oder von Vesikeldispersionen vom ionischen und/oder nichtionischen Typ vorliegt.

## Claims

1. Non-therapeutic cosmetic use of an effective amount of at least one lysate of at least one microorganism of the genus *Bifidobacterium* for preventing and/or reducing skin discomfort sensations on sensitive skin, said lysate being formed from all the intracellular biological constituents and the cell wall and membrane constituents, said skin discomfort sensations being chosen from itching, heating, and stinging and/or tautness sensations,
said lysate being administered topically.

2. Use according to the preceding claim, in which said microorganism lysate is intended for preventing and/or treating the manifestations of dysaesthetic sensations on sensitive skin.

3. Use according to Claim 1 or 2, in which the microorganism of the genus *Bifidobacterium* is chosen from *Bifidobacterium longum, Bifidobacterium bifidum, Bifidobacterium breve, Bifidobacterium animalis, Bifidobacterium lactis, Bifidobacterium infantis, Bifidobacterium adolescent is* or *Bifidobacterium pseudocatenulatum* and mixtures thereof.

4. Use according to any one of Claims 1 to 3, in which the microorganism of the genus *Bifidobacterium* is *Bifidobacterium longum.*

5. Use according to any one of Claims 1 to 4, in which said lysate comprises from 0.1% to 50% by weight, in particular from 1% to 20% by weight of active material(s) derived from the microorganism of the genus *Bifidobacterium.*

6. Cosmetic and/or dermatological composition, which is useful for topically preventing and/or treating a skin disorder on sensitive skin, comprising, in a physiologically acceptable medium, at least an effective amount of at least one lysate of at least one microorganism of the genus *Bifidobacterium,* said lysate being formed from all the intracellular biological constituents and the cell wall and membrane constituents, in combination with at least one compound that is liable to cause skin irritation and also comprising an ancillary probiotic microorganism and/or a lysate of at least one ancillary probiotic microorganism.

7. Composition according to the preceding claim, **characterized in that** said ancillary probiotic microorganism is chosen from ascomycetes such as *Saccharomyces, Yarrowia, Kluyveromyces, Torulaspora, Schizosaccharomyces pombe, Debaromyces, Candida, Pichia, Aspergillus* and *Penicillium,* bacteria of the genus *Bifidobacterium, Bacteroides, Fusobacterium, Melissococcus, Propionibacterium,* Enterococcus, *Lactococcus, Staphylococcus, Peptostreptococcus, Bacillus, Pediococcus, Micrococcus, Leuconostoc, Weissella, Aerococcus, Oenococcus, Lactobacillus* and mixtures thereof.

8. Composition according to either of Claims 6 or 7, **characterized in that** the ancillary microorganism is derived from the group of lactic acid bacteria.

9. Composition according to any one of Claims 6 to 8, **characterized in that** the ancillary probiotic microorganism and/or one of the fractions thereof is present in a proportion of from 0.0001% to 30%, in particular from 0.001% to 15% and more particular from 0.1% to 10% by weight of said composition.

10. Composition according to any one of Claims 6 to 9, **characterized in that** it is in the form of aqueous, aqueous-alcoholic or oily solutions, dispersions of solution type or dispersion of lotion or serum type, emulsions of liquid or semi-liquid consistency of the milk type, obtained by dispersion of a fatty phase in an aqueous phase (O/W) or conversely (W/O), or suspensions or emulsions of soft, semi-solid or solid consistency of the cream type, aqueous or anhydrous gels, or microemulsions, microcapsules, microparticles, or vesicular dispersions of ionic and/or nonionic type.
